# EUROPEAN PATENT APPLICATION

(11) **EP 0 974 323 A1**
(43) Date of publication of application: **26.01.2000**
(21) Application number: 98113667.4
(22) Date of filing: 22.07.1998
(51) Int. Cl.: A61F 13/15

(54) **Apparatus for transporting a continuous web, and for manipulating the web**

(71) Applicant: THE PROCTER & GAMBLE COMPANY, Cincinnati, Ohio 45202 (US)
(72) Inventor: Schmitz, Christoph, 53881 Euskirchen-Stotzheim (DE); Kuntze, Lothar, 53359 Rheinbach (DE)
(74) Representative: Mather, Peter Geoffrey

(57) **Abstract**

The invention provides an apparatus for transporting a continuous web (100), and for manipulating the web, wherein the apparatus comprises a plurality of means for forming web loops (101), each means for forming web loops being positioned between adjacent web support plates (20), each web support plate having an outer web support surface (22), wherein each of the web support surfaces lies in an arc, the arcs lying around the circumference of a circular path, and wherein the web support surfaces and means for forming web loops are rotatably mounted about the axis (25) of the circular path.

The invention also relates to a process for transporting a continuous web (100) around an apparatus (10) whereby the distance between adjacent web support plates (20) is greater than a minimum distance, and wherein the web is transported around the circular path so that a web loop (101) is formed when the distance between adjacent web support plates (20) decreases towards the minimum distance.

## Description

The present invention relates to an apparatus for transporting a continuous web, and for manipulating the web especially to form web loops. The apparatus is particularly useful in the manufacture of disposable absorbent articles, including diapers, adult incontinence products, sanitary napkins and the like.

Manufacturing processes are often required to provide discrete strips of a material onto a continuous web, in such a way that the discrete webs of material are spaced apart along the length of the continuous web. Features manufactured in this way include elastic strips, one example of which is the elastic leg cuffs applied to diapers.

US-A-4 081 301, issued on March 1978, discloses a process for applying a material, such as an elastic material, to a continuous web. The material is only glued along the length of the web in discrete sections, so that when the web is cut the elastic material remains extended only in the discrete section in which it has been glued, and contracts elsewhere.

US-A-4 227 952, issued on 14th October 1980, discloses a conveyor which carries spaced web support plates and wherein a continuous web is tucked in between the support plates to form web loops is known. Various features, such as leg elastic, are then applied to the web which lies on the web support plate, but not to the web loop. This forming process requires less elastic material and provides material savings.

An apparatus of this type comprises a plurality of web support plates, each web support plate having an outer web support surface. However the apparatus is mechanically complex and not well-suited to high speed manufacturing machines.

The object of the present invention is to provide a mechanically simpler and more reliable apparatus for applying features, including elastic features, to a continuous web, in particular on a high speed manufacturing machine.

A further object of the invention is to provide a process for transporting and manipulating a web using the apparatus of the invention.

### Summary of the Invention

The object of the invention is achieved by an apparatus comprising a plurality of means for forming web loops, each means for forming web loops being positioned between adjacent web support plates, wherein each of the web support surfaces lies in an arc, the arcs lying around the circumference of a circular path, and wherein the web support surfaces and means for forming web loops are rotatably mounted about the axis of the circular path. In a preferred embodiment of the invention, the means for forming web loops is provided by rotatably driving the web support plates around the circular path with a circumferential velocity, wherein the circumferential velocity is varied between a minimum circumferential velocity and a maximum circumferential velocity so that the distance between adjacent web support plates varies between a minimum distance and a maximum distance. Preferably the circumferential velocity of the web support plates varies according to a sinusoidal function, and wherein one cycle of the sinusoidal function corresponds to one complete rotation of the web support plate around the circular path.

The invention also relates to a process for transporting a continuous web around the apparatus whereby the distance between adjacent web support plates is greater than the minimum distance, and wherein the web is transported around the circular path so that a web loop is formed when the distance between adjacent web support plates decreases towards the minimum distance.

### Brief Description of the Drawings

Figure 1 shows a diagrammatic representation of a cross-section through the apparatus of the present invention.
Figure 2 shows a more detailed cross-section through the apparatus of the present invention, including linkages between adjacent web support plates.
Figure 3 shows a representation in perspective of the path of a continuous web as it passes around the apparatus of the present invention in a first embodiment of the process of the invention.
Figure 4 shows a representation in perspective of the path of a continuous web as it passes around the apparatus of the present invention in a second embodiment of the process of the invention.
Figure 5 shows a representation in perspective of the path of a continuous web as it passes around the apparatus of the present invention in a third embodiment of the process of the invention. Figure 5a shows the profile of a curved elastic feature applied to the continuous web.

### Detailed Description of the Invention

It will be readily apparent to those skilled in the art that although the following description of the present invention is in connection with a single use diaper structure having discrete elastic regions or strips, the present invention may be practiced with equal facility on nearly any web.

In the following description a "continuous web" is a web of material which is continuous in the machine direction. A preferred continuous web comprises a plurality of interconnected single use disposable absorbent articles, such as diapers: Typically, each diaper is comprised of an absorbent pad element or absorbent core, and elastomeric elements or patches. The absorbent pad elements and the elastomeric elements are located between a backsheet and a topsheet, or alternatively, on top of a backsheet or topsheet. The continuous webs of backsheet material and topsheet material are preferably maintained under very slight tension in the machine direction to prevent wrinkling and to facilitate registration with the diaper assembly and converting operations until the completed diaper web is severed into discrete diapers by cutting across the width of the web.

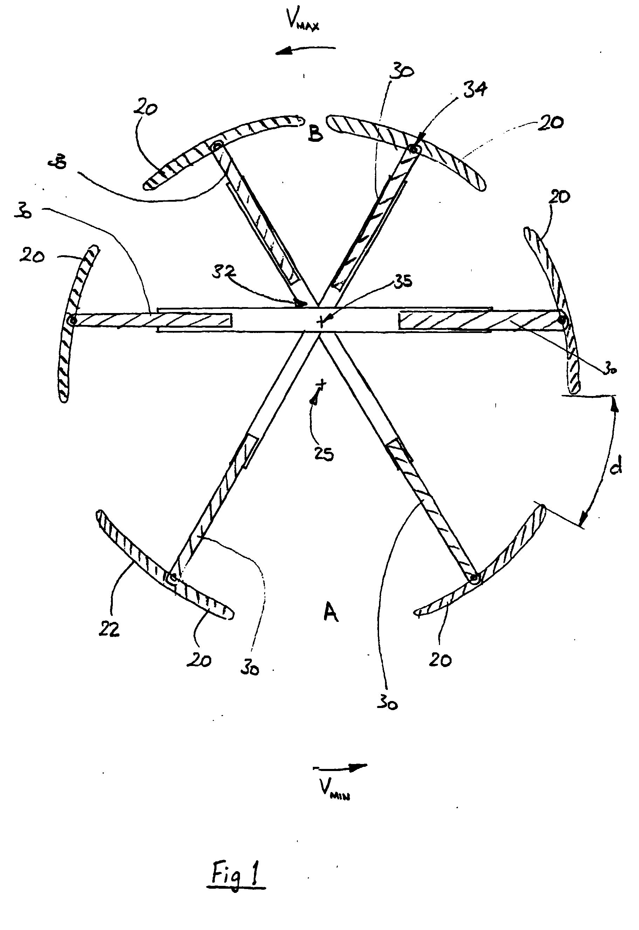

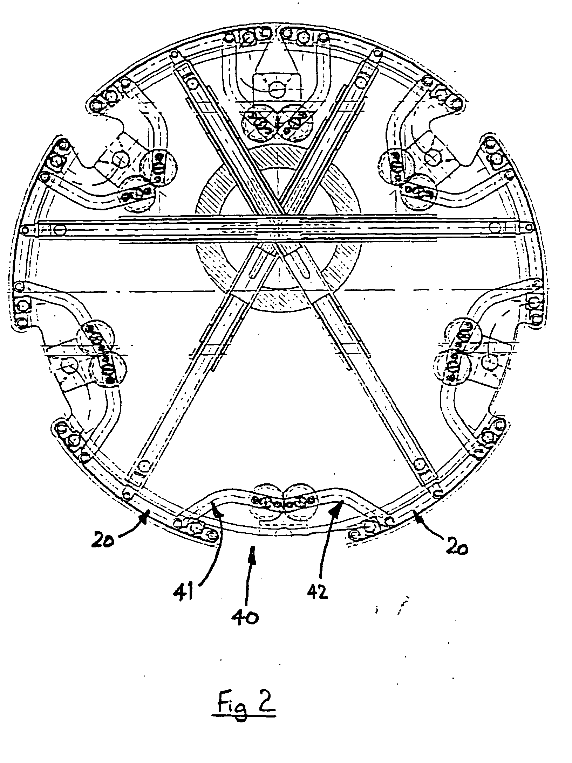

Figures 1 and 2 illustrate a cross-section through a preferred embodiment of the apparatus of the present invention. An apparatus having six web support plates 20 is illustrated, each web support plate comprises a web support surface 22 facing outwardly, and the web support plates 20 are rotated so that the web support surfaces 22 trace out an essentially circular path. The apparatus will now be described with reference to one of the web support plates as it moves around the circular path illustrated in Figure 1. An anti-clockwise rotation is illustrated, but the invention could be equally well put into practice with a clockwise rotation. As the web support plate 20 passes through point A of the circular path (at the bottom of the circular path as illustrated in Figure 1), it has a minimum circumferential velocity Vₘᵢₙ. As the web support plate 20 is rotated towards the top of the circular path it is accelerated until it reaches a maximum circumferential velocity Vₘₐₓ at point B of the circular path. As the web support plate 20 continues around the circular path it slows down until it returns to point A. Adjacent web support plates are spaced apart by a distance d. The adjacent web support plates either side of point A in Figure 1 have a maximum distance d between them. As the web support plates are rotated one of the web support plates has a faster circumferential velocity than the adjacent web support plate, and the faster web support plate catches up with the slower web support plate, thereby reducing the distance d between them. When the adjacent web support plates lie either side of point B in Figure 1 the distance between them is a minimum. It is preferred that the circumferential velocity V of the web support plates 20 varies according to a sinusoidal function, and wherein one cycle of the sinusoidal function corresponds to one complete rotation of the web support plate 20 around the circular path.

Consequently a continuous web 100 placed against the web support plates is transported around the apparatus and furthermore the continuous web 100 is manipulated in such a way that a web loop 101 is formed between adjacent web support plates 20 as the adjacent web support plates 20 move closer together.

In a particularly preferred embodiment of the invention the circumferential velocity of the web support plates is controlled by mechanical means. In Figure 1 the mechanical means comprises six extendible arms 30, that is to say one extendible arm 30 to drive each of the web support plates 20. Each extendible arm 30 has a proximal end 30 and a distal end 34, the proximal end 32 of each extendible arm 30 being mounted on a second axis of rotation 35 and the distal end 34 of each extendible arm 30 being pivotally mounted on each web support plate 20. The principal axis 25 and the second axis 35 are parallel but off-set in relation to each other, so that the extendible arms 30 drive the web support plates 20 around the circular path with the variable circumferential velocity.

Figure 2 shows a similar schematic representation to Figure 1, and additionally Figure 2 also illustrates preferred linking means 40. Adjacent web support plates 20 are pivotally connected to each other by linking means 40. The linking means illustrated comprise a first link 41 and a second link 42. The first link 41 and the second link 42 are pivotally connected to each other near one end of the links, the other end of each link being pivotally connected near to the ends of adjacent web support plates 20.

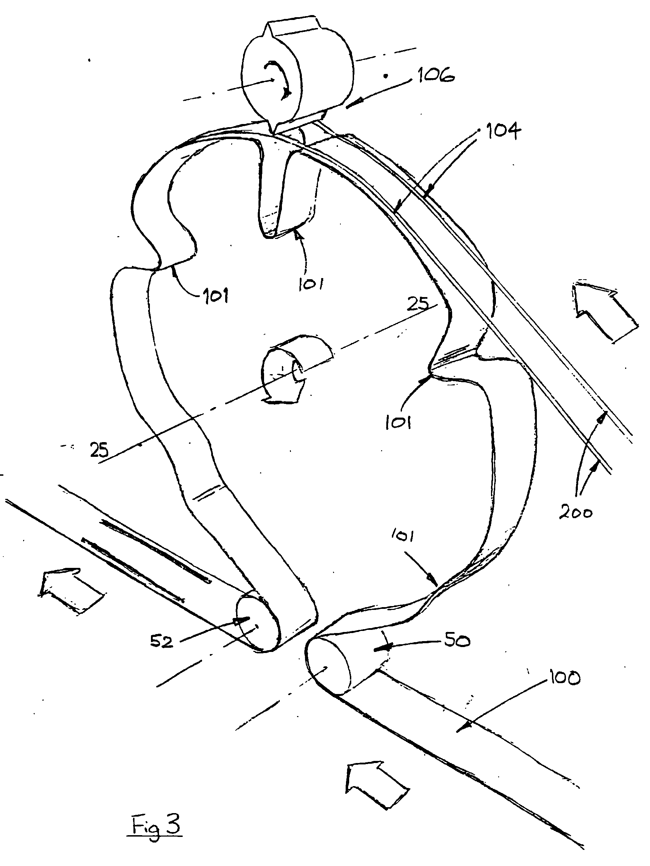

Figure 3 illustrates the path of a continuous web 100 as it passes around the apparatus of the present invention in a first embodiment of the process of the invention. The web is fed to a transfer roll 50 by a feeding means (not shown) and is transferred to the web support surface of the apparatus. The apparatus itself is not shown in Figure 3 for clarity. As the web is drawn around the circular path by means of the web support plates (not shown), web loops 101 are formed as described hereinabove. Figure 3 also shows a pair of elastic strips 200 being fed by a feeding means (not shown) to application points 104. At the application points 104 the elastic strips 200 are attached to the continuous web 100. The means of attachment is not important in this invention and may be achieved by glue, hot melt, self-adhesive material or any other means. Figure 3 also shows a cutting station 106 at which the elastic strips 200 are cut into discrete strips. The continuous web 100 is not necessarily cut at the cutting station 106, indeed it is preferred that the continuous web 100 is not cut on the apparatus of the present invention. The continuous web 100 is not cut because the cutting station 106 is adjacent to a web loop 101 as shown. Subsequently the web 100 accelerates around the apparatus, thereby releasing the web loops 101, and the continuous web with discrete lengths of elastic material adhered to it, the discrete lengths of elastic material being spaced apart, is peeled off the web support surface at a second transfer roll 52.

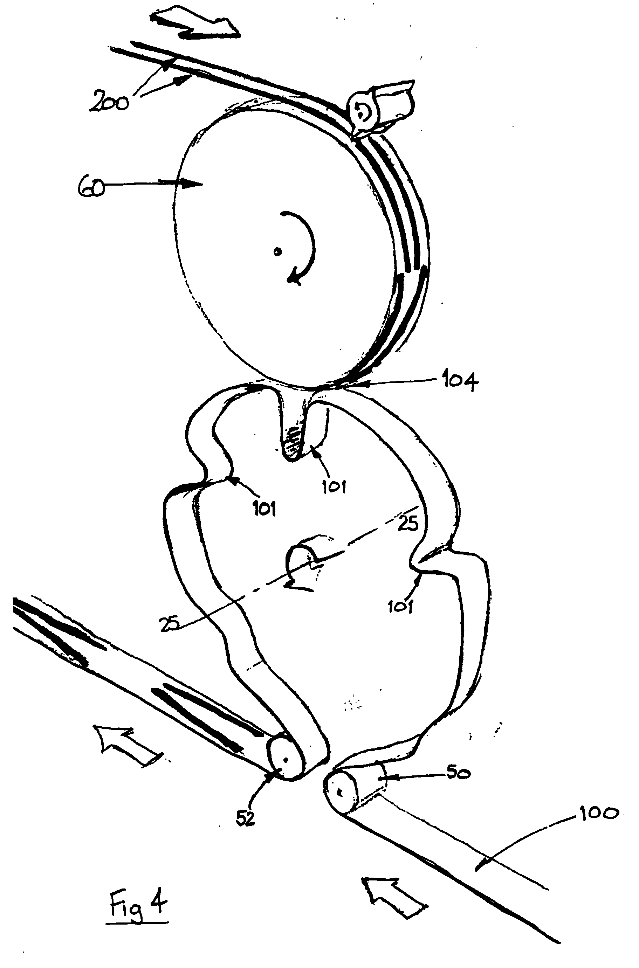

Figure 4 illustrates the path of a continuous web 100 as it passes around the apparatus of the present invention in a second embodiment of the process of the invention. The web is fed to a transfer roll 50 by a feeding means (not shown) and is transferred to the web support surface of the apparatus. The apparatus itself is not shown in Figure 4 for clarity. As the web is drawn around the circular path by means of the web support plates (not shown), web loops 101 are formed as described hereinabove.

In Figure 4 the elastic strips 200 are provided on the surface of an applicator roll 60. The elastic strips 200 are cut and deflected on the surface of the applicator roll 60 prior to the application point 104.

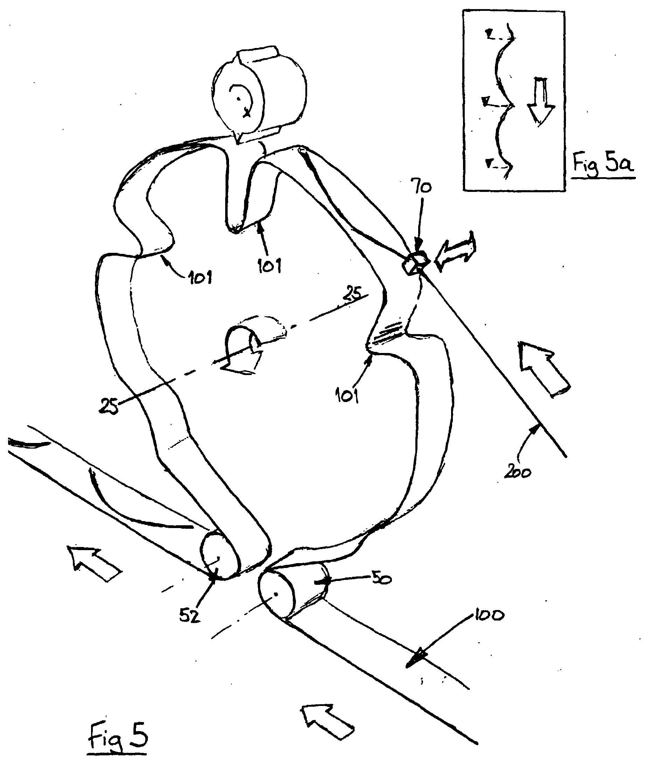

Figure 5 illustrates the path of a continuous web 100 as it passes around the apparatus of the present invention in a third embodiment of the process of the invention. The web is fed to a transfer roll 50 by a feeding means (not shown) and is transferred to the web support surface of the apparatus. The apparatus itself is not shown in Figure 5 for clarity. As the web is drawn around the circular path by means of the web support plates (not shown), web loops 101 are formed as described hereinabove.

In Figure 5 a deflection means 70 is provided in order to apply an elastic strip 200 in a curved profile. The deflection means 70 is adjacent to the application point 104 moves in the cross-machine direction with a frequency corresponding to the speed of the cycle of the web support plates 20.

Figure 5a shows the profile of a curved elastic feature applied to the continuous web.

## Claims

1. An apparatus (10) for transporting a continuous web (100), and for manipulating the web (100), the apparatus comprising a plurality of web support plates (20), each web support plate (20) having an outer web support surface (22), the apparatus further comprising a means for forming web loops (101) between adjacent web support plates (20), characterised in that each of the web support surfaces (22) lies in an arc, each of the, arcs lying around the circumference of a circular path, and wherein the web support surfaces (22) and means for forming web loops (101) are rotatably mounted about the principal axis (25) of the circular path.

2. An apparatus (10) according to claim 1 wherein the means for forming web loops (101) is provided by rotatably driving the web support plates (20) around the circular path with a circumferential velocity (V), wherein the circumferential velocity (V) is varied between a minimum circumferential velocity (Vₘₐₓ) and a maximum circumferential velocity (Vₘᵢₙ) so that the distance (d) between adjacent web support plates (20) varies between a minimum distance and a maximum distance.

3. An apparatus (10) according to claim 2 wherein the circumferential velocity (V) of the web support plates (20) varies according to a sinusoidal function, and wherein one cycle of the sinusoidal function corresponds to one complete rotation of the web support plate (20) around the circular path.

4. An apparatus (10) according to claim 2 wherein each web support plate (20) is driven around the circular path by an extendible arm (30), the proximal end (32) of each extendible arm (30) being mounted on a second axis of rotation (35) and the distal end (34) of each extendible arm (30) being pivotally mounted on each web support plate (20), and wherein the principal axis (25) and the second axis (35) are parallel but off-set in relation to each other, so that the extendible arms (30) drive the web support plates (20) around the circular path with the variable circumferential velocity (V).

5. An apparatus (10) according to any of claims 1 to 4 wherein adjacent web support plates (20) are pivotally connected to each other by linking means (40).

6. A process for transporting a continuous web (100), and for manipulating the web, wherein the web is placed against the web support plates (20) of the apparatus (10) of any of claims 1 to 5 at a position where the distance (d) between adjacent web support plates (20) is greater than the minimum distance, and wherein the web (100) is transported around the circular path so that a web loop (101) is formed when the distance (d) between adjacent web support plates (20) decreases towards the minimum distance.

7. A process according to claim 6 for applying a material (200) to the continuous web (100) wherein the material (200) is applied to the web (100) at an application point (104) which is adjacent to the circular path of the web support plates (20), so that the material (200) is applied to that part of the web lying upon one of the web support plates (20), but the material (200) is not applied to the remaining part of the web which is in the web loop (101).

8. A process according to claim 7 wherein the material is cut at a cuffing station (106) so that the material (200) remains attached to the continuous web (100) only in discrete sections, each section corresponding to the part of the web lying adjacent to the web support plate (20).

9. A process according to either of claims 7 or 8 wherein the material (100) is an elastic material, preferably an elastic material applied under tension at the application point (104).
